(19) Europäisches Patentamt
European Patent Office
Office européen des brevets

(11) Publication number: **0 595 226 A2**

# (12) EUROPEAN PATENT APPLICATION

(21) Application number: **93117209.2**

(22) Date of filing: **22.10.93**

(51) Int. Cl.5: **A61K 47/48**, C08F 220/28, C08F 212/14

A request for correction letter dated 27.01.94 (received 28.01.94) translation of prior doc. + fig. 2 has been filed pursuant to Rule 88 EPC. A decision on the request will be taken during the proceedings before the Examining Division (Guidelines for Examination in the EPO, A-V, 2.2).

(30) Priority: **30.10.92 JP 293362/92**
**27.04.93 JP 101247/93**

(43) Date of publication of application:
**04.05.94 Bulletin 94/18**

(84) Designated Contracting States:
**DE FR GB**

(71) Applicant: **HOYA CORPORATION**
**7-5, Naka-Ochiai 2-Chome**
**Shinjuku-ku Tokyo(JP)**

(72) Inventor: **Imafuku, Suguru**
**135, Hachimanyama,**
**Kodama-machi**
**Kodama-gun, Saitama-ken, 367-02(JP)**
Inventor: **Iwamoto, Hidetoshi**
**1263-9, Oaza Shichihongi,**
**Kamisato-machi**
**Kodama-gun, Saitama-ken, 369-03(JP)**
Inventor: **Yamauchi, Aizo**
**2-7-3-206, Honjo,**
**Honjo-shi**
**Saitama-ken, 367(JP)**

(74) Representative: **Türk, Gille, Hrabal, Leifert**
**Brucknerstrasse 20**
**D-40593 Düsseldorf (DE)**

## (54) Active substance-containing polymer gel.

(57) An active substance-containing polymer gel characterized by comprising an active substance with an anionic substituent thereon, held by a copolymer gel through adsorption therein, said copolymer gel being prepared by copolymerization of a monomer mixture containing at least a hydrocarbon group-containing (meth) acrylate which has one or more hydroxyl groups and has optionally an intramolecular ether linkage, and a monomer with a quaternary ammonium salt on the side chain is capable of strongly holding in the copolymer gel, the anionic functional group-containing active substance to produce a significant sustained release effect and of being worked into any desired form in an extremely easy manner.

FIG. 1

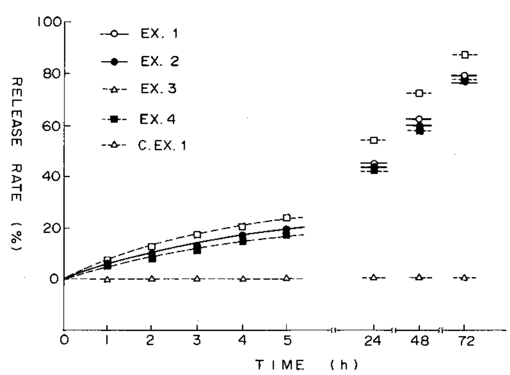




Rank Xerox (UK) Business Services
(3.10/3.09/3.3.4)

## Field of the Invention

The present invention relates to an active substance-containing polymer gel, and more particularly it is concerned with an active substance-containing polymer gel particularly useful for drug delivery systems (DDS).

The active substance-containing polymer gel according to the present invention may be used for soft contact lens and DDS for application to ophthalmic mucous membrane (e.g., trade name "Ocusert", manufactured by FUJISAWA PHARMACEUTICAL CO., LTD. in Japan) etc. with advantages.

## Description of the Prior Art

In view of its elasticity resembling the living body system, the hydrogel composed of a polymer of a three-dimensional network structure swelled with an aqueous solvent has been earnestly investigated for development of medical materials. Representative embodiments thereof include soft contact lens consisting mainly of polyhydroxyethyl methacrylate which produces an extremely excellent feeling of insertion (wearing) due to its elasticity like the living body system.

Conception of drug delivery system (DDS) was presented around 1970 for establishing the optimum dose and the best administration process for drugs under consideration of their pharmacokinetics in the body, and recently researches are being made for development of more functional DDS in a wide field of technologies.

An example of process for the control of drug delivery is described in Japanese Patent Laid-Open SHO 52-56148. In brief, according to the example, polyvinyl alcohol (hereunder referred to as "PVA") prepared as an aqueous solution or in the state swelled with water or hydrated gel of crosslinked PVA prepared in advance is treated by radiation of active rays in the presence of a radical polymerizable monomer which has a electrolyzable group such as carboxyl group in the form of salt, sulfonate group in the form of salt, phosphoric acid group in the form of salt, a group of quaternary ammonium salt or the like, or a polar group such as amino group, carbonyl group, sulfone group, nitro group, etc. so as to prepare hydrated gel of PVA which has been graft polymerized with the monomer having an electrolyzable or polar group, after which the gel is impregnated with an active substance such as drug to provide an active substance-containing polymer gel. This device aims to retard the delivery of the active substance by incorporating it into the network structure of the crosslinked PVA to form a weak bond between the functional groups of the active substance and the electrolyzable or polar groups of the PVA molecules.

However, the use of extremely special $\gamma$ rays as the active rays for the cross-linking of the starting PVA which is involved in the preparation of the active substance-containing polymer gel as mentioned above is not general cross-linking means available for use in usual processes for the preparation of polymer gels. Furthermore, in the above active substance-containing polymer it is necessary to use an increased dose of $\gamma$ rays for the lowered delivery rate of the active substance, and thus in the polymer gel of the prior art it is impossible to improve the sustained release while keeping its strength because it tends to reduce the mechanical strength due to the increased cross-linking density and occurrence of side reactions.

## Summary of the Invention

The present invention has been accomplished to solve the above problems of the prior art, and it aims to provide an active substance-containing polymer gel which enables strongly to maintain therein the active substance which has anionic functional groups, for its significant sustained release and which can be extremely easily formed into any desired shape.

The present invention has been completed to attain the above aim. A characteristic aspect of the present invention resides in an active substance-containing polymer gel characterized by comprising an active substance having an anionic substituent held by a copolymer gel through adsorption therein, said copolymer gel being prepared by copolymerization of a monomer mixture containing at least a hydrocarbon group-containing (meth)acrylate which has one or more hydroxyl groups and has optionally an intramolecular ether linkage; and a monomer with a quaternary ammonium salt on the side chain (said active substance-containing polymer gel being hereunder referred to as "active substance-containing polymer gel A").

Another characteristic aspect of the invention is in an active substance-containing polymer gel characterized by comprising an active substance having an anionic substituent, held by a copolymer gel prepared through adsorption therein, said copolymer gel being prepared by copolymerization of a monomer mixture containing at least a hydrocarbon group-containing (meth)acrylate which has one or more hydroxyl

groups and has optionally an intramolecular ether linkage; a monomer with a quaternary ammonium salt on the side chain; and an alkyl or a fluoroalkyl group-containing (meth)acrylate (said active substance-containing polymer gel being hereunder referred to as "active substance-containing polymer gel B").

## Brief Description of the Drawings

Fig. 1 is a graph showing the change with time in the rate of the orange II released from the hydrogels of Examples 1-4 and Comparative Example 1;
Fig. 2 is a graph showing the change with time in the rate of the water-soluble azulene released from the hydrogels of Examples 5-8 and Comparative Example 2;
Fig. 3 is a graph comparing the change with time in the rate of the DSCG released from the hydrogels prepared in Examples 1 and 9-10;
Fig. 4 is a graph comparing the change with time in the rate of the DSCG released from the hydrogels prepared in Examples 1 and 11-12; and
Fig. 5 is a graph comparing the change with time in the rate of the DSCG released from the hydrogels prepared in Examples 1 and 13-14.

## Detailed Description of the Invention

Both active substance-containing polymer gels A and B according to the present invention are copolymer gels with an active substance adsorbed therein, and accordingly the explanation will first be made of the copolymer gel, and then of the active substance.

### Copolymer gel

The hydrocarbon group-containing (meth)acrylate which has one or more hydroxyl groups and has optionally, an intramolecular ether linkage (said (meth)acrylate being hereunder referred to as "H(M)A"), which is a monomer constituting the copolymer gel in active substance-containing polymer gel A according to the present invention, includes 2-hydroxymethyl (meth)acrylate, 2-hydroxyehtyl (meth)acrylate, 2-hydroxypropyl (meth)acrylate, 2,3-dihydroxypropyl (meth)acrylate, 2-polyethyleneglycol monoacrylate, etc., and further a mixture of two or more thereof. Here, the (meth)acrylate includes both acrylate and methacrylate throughout the specification. Of them 2-hydroxyethyl methacrylate (hereunder referred to as "HEMA") is preferable. This is because the workability of a polymer of HEMA is excellent, and it is easy to adjust the moisture content of the hydrogel prepared when using it as a comonomer.

Another monomer which is a component of the copolymer gel constituting active substance-containing polymer gel A of the present invention, is a monomer with a quaternary salt on the side chain, which is essential to attain a significant sustained release effect due to strong holding of the anionic group-having active substance to the hydrogel by the ionic interaction between the anionic group in the active substance and the quaternary ammonium ion in the hydrogel.

As the monomer with a quaternary ammonium salt on the side chain which is used for the preparation of active substance-containing polymer gel A of the present invention (said monomer being hereunder referred to as "tert-Am monomer"), preferred are a vinylbenzyl trialkyl ammonium salt represented by the following general formula (I) or ethyl (meth)acrylate represented by the following general formula (II):

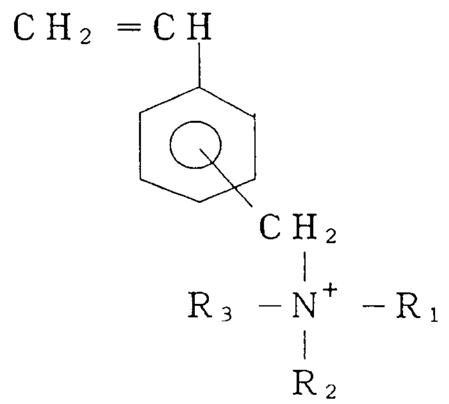

(I)

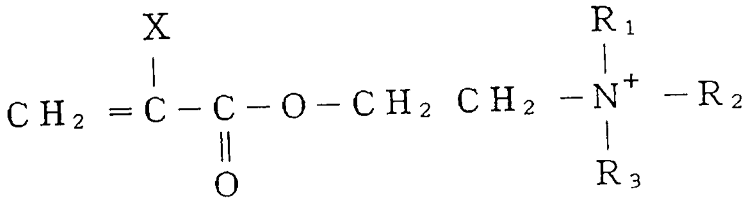

(II)

In the above general formulae (I) and (II), X is a hydrogen atom or a methyl group, $R_1$ is a $C_5$-$C_{12}$ alkyl group, $R_2$ and $R_3$ are the same or different $C_1$-$C_2$ alkyl groups, or $R_1$, $R_2$ and $R_3$ are the same or different $C_1$-$C_4$ alkyl groups. That is, among the three alkyl groups in the general formulae (I) and (II), when one is a long chain alkyl group of 5-12 carbon atoms, then the other two must be very short alkyl groups of 1 or 2 carbon atoms, whereas, all the three alkyl groups may have 1-4 carbon atoms, respectively, if they are very short alkyl groups each consisting of 4 or less carbon atoms.

Illustrative embodiments of the vinylbenzyl trialkyl ammonium salt represented by the general formula (I) are, e.g., vinylbenzyl trimethyl ammonium salt (particularly ammonium chloride), vinylbenzyl triethyl ammonium salt (particularly ammonium chloride), vinylbenzyl dimethyl ethyl ammonium salt (particularly ammonium chloride), vinylbenzyl dimethyl isopropyl ammonium salt (particularly ammonium chloride), vinylbenzyl n-butyl dimethyl ammonium salt (particularly ammonium chloride), vinylbenzyl dimethyl pentyl ammonium salt (particularly ammonium chloride), etc.

Ethyl (meth)acrylate represented by the general formula (II) includes, for example, 2-methacryloxyethyl trimethyl ammonium salt (particularly ammonium chloride), 2-methacryloxyethyl dimethyl ethyl ammonium salt (particularly ammonium chloride), 2-methacryloxyethyl dimethyl n-pentyl ammonium salt (particularly ammonium chloride), 2-acryloylethyl trimethyl ammonium salt (particularly ammonium chloride), 2-acryloylethyl dimethyl ethyl ammonium salt (particularly ammonium chloride), 2-acryloylethyl triethyl ammonium salt (particularly ammonium chloride), 2-acryloylethyl dimethyl n-pentyl ammonium salt (particularly ammonium chloride), etc.

The amounts of H(M)A and tert-Am monomers to be used is preferably in the range of $0.001 \leqq F \leqq 0.05$ wherein the monomer composition ratio $F = ([C]/([H]+[C])$, [C] = the molar concentration of tert-Am monomer, and [H] = the molar concentration of H(M)A. Most preferred is $0.005 \leqq F \leqq 0.02$. At ratios below 0.001 the amount of the active substance to be held is too small due to the short supply of [C] with respect to [H] in the hydrogel, whereas the mechanical strength may lower at ratios over 0.05.

A crosslinkable monomer may be used as a component of the copolymer gel together with the above mentioned essential components for the preparation of active substance-containing polymer gel A according to the present invention. The purpose of the use of the crosslinkable monomer is to facilitate the formation of the network structure of the hydrogel and to improve of the mechanical strength of the gel, and embodiments thereof include bismethylene acrylamide, ethylene glycol dimethacrylate (hereunder abbreviated to EDMA), 2-hydroxy-1,3-dimethacryloxypropane, trimethylolpropane triacrylate, etc. The amount of the crosslinkable monomer to be used is preferred to be 0.1-10 mol% of the total monomers. Most preferable is 0.1-3 mol%.

At less than 0.1 mol % the shortage of the crosslinkable monomer tends to cause poor form retention of the hydrogel, while in case of the amount exceeding 10 mol%, increased crosslinked points may cause

brittleness of the gel which leads to its lowered mechanical strength, resulting in poor mechanical processability in some cases.

As optional components of the copolymer gel of active substance-containing polymer gel A, a hydrophilic monomer and/or a hydrophobic monomer may be used. The hydrophilic and hydrophobic monomers have an effect to adjust the moisture content of the resulting hydrogel, and further serve to adjust the amount of the active substance to be adsorbed in the hydrogel.

As the hydrophilic monomer any one is available for use which has biocompatibility and excellent compatibility with H(M)A and tert-Am monomer, and preferable embodiments thereof include, for example, N,N-dimethylacrylamide, N,N-dimethylmethacrylamide, vinylpyrrolidone, etc.

Also as the hydrophobic monomer any one is available for use which has biocompatibility and excellent compatibility with H(M)A and tert-Am monomer, and preferably is, e.g., methacrylic acid, methyl methacrylate, isobutyl methacrylate, 2,2,2-trifluoroethyl methacrylate or the like.

As mentioned above, in the copolymer gel used in the active substance-containing polymer gel A, H-(M)A and tert-Am monomer are essential components, and a hydrophilic monomer and/or a hydrophobic monomer are optional components of the copolymer gel. On the other hand, the copolymer gel constituting active substance-containing polymer gel B is different from that of active substance-containing polymer A only in that the former contains another essential (meth)acrylate having an alkyl group or a fluoroalkyl group in addition to H(M)A and tert-Am monomers discussed above. Therefore, further explanation will now be given with reference only to the (meth)acrylate having an alkyl group or fluoroalkyl group.

The (meth)acrylate having an alkyl group or a fluoroalkyl group, which is a monomer component constituting the copolymer gel composing the active substance-containing polymer gel B according to the present invention, has functions of adjusting the swelling coefficient and water content of the hydrogel and of controlling the release rate of the active substance with an anionic substituent. The (meth)acrylate having an alkyl or a fluoroalkyl group (hereunder referred to as R(M)A) is preferred to be one represented by the following general formula (III).

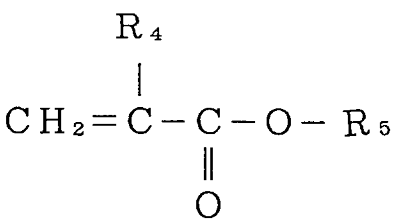
(III)

In the above general formula (III), $R_4$ is a methyl group or a hydrogen atom, and $R_5$ is a hydrogen atom or a $C_1$-$C_{12}$ alkyl group which may be substituted with fluorine atom(s).

Concretely, the R(M)A represented by the general formula (III) includes, for instance, methacrylic acid, methyl (meth)acrylate, ethyl (meth)acrylate, n-propyl (meth) acrylate, isopropyl (meth)acrylate, n-butyl (meth)acrylate, isobutyl (meth)acrylate, tert-butyl (meth)acrylate, n-hexyl (meth)acrylate, n-octyl (meth)-acrylate, n-heptyl (meth) acrylate, n-nonyl (meth)acrylate, 2,2,2-trifluoroethyl (meth)acrylate, 2,2,3,3-tetrafluoropropyl (meth)acrylate, 2,2,3,3,3-pentafluoropropyl (meth)acrylate, 1,1,1,3,3,3-hexafluoroisopropyl (meth)acrylate, etc.

The amount of R(M)A to be added is preferably 1-30 mol% of the total monomers. Most preferably is 3-25 mol%. At a proportion less than 1 mol %, the release rate of the active substance which has an anionic substituent cannot be controlled due to shortage of R(M)A. On the other hand, in some cases, the swelling coefficient and water content of the hydrogel decrease remarkably at a proportion more than 30 mol%, and accordingly the anionic substituent-containing active substance may become hard to be released.

The H(M)A, R(M)A and tert-Am monomer referred to above are desirably used in such amounts as to satisfy $0.001 \leqq F \leqq 0.1$ wherein the monomer composition ratio (F) = ([C]/([H]+[R]+[C]), and [C], [H] and [R] signify the molar concentrations of tert-Am monomer, H(M)A and R(M)A, respectively. Particularly preferred is $0.005 \leqq F \leqq 0.05$. At values less than 0.001, [C] comes short for [H] and [R] in the hydrogel, leading to a decreased amount of the active substance held thereby. On the other hand the mechanical strength of the resulting hydrogel may lower at values over 0.1.

Next, explanation will be given regarding the active substance in connection with active substance-containing polymer gels A and B according to the present invention.

Active substance

The active substance held in the copolymer gel mentioned above via adsorption includes any one having an anionic substituent selected from those having an intramolecular carboxylic acid group, sulforic acid group, phenolic hydroxy group or a salt thereof. Illustrative embodiments of the active substance which produces a satisfactory pharmacological effect when the polymer gel is used for an ophthalmic application, for example, disodium prednisolone-21-phosphate, disodium dexamethasone-21-phosphate, dexamethasone-21-metasulfobenzoate sodium salt, sodium dexamethasone-21-sulfate (adrenal cortical hormone preparation), sodium sulbenicillin, sodium carbenicillin (antibiotic), dipotassium glycyrrhizinate, sodium guaiazulenesulfonate (nonsteroidal anti-inflammatory agent), disodium cromoglycate (antiallergic agent), glutathione, Catalin (a remedy for cataract), N-acetylcysteine (an accelerator for cornea cure), etc.

Active substance-containing polymer gels A and B of the present invention may be utilized in various uses in addition to the ophthalmic application. Such uses include various drug delivery systems (DDS), bactericidal sheet, insecticidal sheet, fomentation and so on, and the active substance is selected appropriately depending on the intended use.

As an example of using the active substance-containing polymer gel of the present invention, when it is intended to use for an ophthalmic application, e.g., a contact lens, wearing of a soft contact lens made of the active substance-containing polymer gel of the present invention impregnated with an anti-inflammatory agent or the like over the eye will be expected to cure the eye if the eye suffers from inflammation, etc.

In the other fields of medicine, a plaster impregnated with glyceryl trinitrate, a remedy for angina pectoris; and a plaster impregnated with scopolamine, a motion sickness-preventing medicine, etc. are well known and have been put to practical use. In addition, plasters have become the targets of earnest investigation which enable the supply of an medicine at a constant concentration which is hard to be kept at a constant blood concentration via oral administration, such as one for diabetes mellitus, elevated blood pressure, climacteric disturbance or the like and further a sudden increase in the blood concentration of which may involve some risk to the patient.

The active substance-containing polymer gel of the present invention is extremely useful for the production of "percutaneous preparations" intended for administration of drugs expected to be released gradually as mentioned above.

Meanwhile, in domestic closets, etc. molds are apt to grow due to moisture or the like, and also insects tend to grow which live on the fibers of clothes. As means to prevent the growth of such molds and insects over a long period of time now commercially available are sheets impregnated with fungicidal or insecticidal agents, which have become to be usually used for various purposes.

Even fungicides and insecticides may have a bad influence upon or are even poisonous to humans if they are present at high concentrations in the air, but this problem is overcome by enclosing them in an active substance-containing polymer gel according to the present invention which releases them little by little, thereby providing a prolonged fungicidal or insecticidal effect at a concentration in the air which is safe to humans.

As mentioned above, the active substance-containing polymer gels provided by the present invention may be applied to various cases in daily life as well.

A further explanation will now be made regarding processes for the preparation of active substance-containing polymer gels A and B of the present invention with reference to the case of an ophthalmic application.

For the preparation of a hydrogel for an ophthalmic application, first a polymerization initiator is added to the mixtures of the monomers mentioned above which is then stirred well to produce a homogenous solution of the monomer mixture.

Here, the radical initiator available for use may be any conventional one, for example, a peroxide such as lauroyl peroxide, cumene hydroperoxide, benzoyl peroxide or the like, azobisvaleronitrile or azobisisobutylonitrile (hereunder abbreviated to AIBN), of which desirable is AIBN. The amount of the initiator to be used is preferred to be in the order of 0.02-0.5 mol% of the total monomers.

Next, the above mixture solution is placed in a metal, glass or plastic container of any desired shape, sealed and heated stepwise or continuously to a temperature range of 25-120 °C in a thermostat, etc. for the completion of polymerization in 12-120 hours. The polymerization may also be accomplished by photopolymerization with ultraviolet rays, visible rays or the like. Further, the above monomer mixture solution may be mixed with an organic solvent for solution polymerization. Desired embodiments of the organic solvent include, for example, methanol, ethanol, acetone, etc.

After polymerization, the mixture is cooled to room temperature, and the polymerization product is cut and polished to obtain a desired shape. The desired shape is, for example, an approximate form of the

conventional contact lens or any other ophthalmic insertable products, film form, sheet form, etc. In addition, the cutting and polishing become unnecessary if the polymerization procedure is carried out in a container having the corresponding shape to a shape of a contact lens.

Thereafter, the polymerization product having a desired shape is hydrated for swelling to obtain a hydrogel. The liquid to be used for hydration to swelling (hereunder referred to as "hydration liquid") is, for example, water, physiologic saline, isotonic buffer solution or the like. The hydration liquid is heated for immersion of the above polymerization product therein under conditions of a temperature of 60-100 °C and the ambient pressure, upon which the product rapidly undergoes a change into hydrated, swelled state.

The above treatment for hydration to swelling may simultaneously remove the residual monomers remaining in the polymerization product.

Next, a solution of an active substance with an anionic substituent is prepared in advance, into which the above hydrogel is immersed to impregnate the latter with the active substance, thereby providing a hydrogel in which an active substance is adsorbed therein.

The solvent available for dissolving the above active substance is water, hydrophilic solvent or a mixed solvent of water and a hydrophilic solvent. Here, the hydrophilic solvent includes an alcohol such as methanol, ethanol, isopropanol or n-butanol, dimethylsulfoxide or the like, while the mixed solvent of water and a hydrophilic solvent includes one of water and an alcohol or water and dimethylsulfoxide or the like.

The concentration of the active substance to be contained in the above solution is determined appropriately depending on the respective active substances in consideration of the solubility of the active substance, its minimum effective concentration required for producing the expected pharmaceutical efficacy, its maximum safety concentration, etc., and usually a concentration of 1.0 x $10^{-6}$ (mol/l) to 1.0 x $10^{-2}$(mole/l) is preferred.

## Examples

The present invention will now be explained in more detail with reference to the Examples, without limiting it thereto.

### Example 1 (Active substance-containing polymer gel A)

In a 50 ml ampoule there were placed 32.2 g (0.247 mol) of HEMA, 0.53 g (0.0025 mol) of a vinyl benzyl trimethyl ammonium salt (hereunder abbrev. as QBm), 0.25 g (0.5 mol% of the total monomers) of EDMA, and 0.033 g (0.08 mol% of the total monomers) of AIBN, and the mixture was stirred for 1 hour in a nitrogen atmosphere. Here, the monomer composition ratio F was 0.01.

After the stirring, the mixture was transferred to a polyethylene container (d.: 15 mm, h.: 17 mm), and then subjected to polymerization at 50-100 °C for 72 hours. The resulting polymer was taken out of the container and cut into flat disks with a diameter of 13 mm and a thickness of 0.5 mm, after which the surface was polished to provide flat transparent disks. The disks were immersed in distilled water at 80 °C for 2 hours for swelling due to hydration, thereby producing a hydrogel from which the residual monomers were removed.

To 10 ml of a 20% aqueous solution of ethanol was added 0.35 mg (1 x $10^{-6}$ mol) of sodium 4-[(2-hydroxy-1-naphthalyl)azo]-benzenesulfonate (another name: acid orange 7; hereunder abbrev. to orange II), a dye comprising a sulfonate ion used as the test substance, and then the mixture was stirred to prepare 10 ml of a solution of orange II in ethanol. The hydrogel was placed in the orange II solution for a 24 hours immersion at 25 °C to adsorb the orange II in the hydrogel. Thereafter, the hydrogel with the orange II adsorbed therein was immersed into 50 ml of distilled water at 25 °C for 48 hours to replace the ethanol by the distilled water while releasing the free orange II not combined with the cations in the hydrogel.

### Measurement of the amount of the orange II adsorbed in the hydrogel

As mentioned above, the hydrogel was immersed in 10 ml of a solution of orange II in ethanol (1.0 x $10^{-4}$ mol/l) for 24 hours; and the adsorption was confirmed to have attained the equilibrium. Thereafter, in order to measure the amount of the orange II adsorbed in the hydrogel, measurement of light absorption at a wavelength of 486.6 nm was made of the residual solution of orange II in ethanol after the 24 hours immersion (liquid A) and of the distilled water which contained the free orange II released from the orange II-containing hydrogel after immersing in distilled water for 48 hours (liquid B), to determine the orange II content each of the liquids A and B. The difference was calculated to determine the amount of the orange II combined with the cations in the above hydrogel. The spectrophotometer used was Model U-3210 of

Hitachi Ltd. in Japan .

In this example, the amount of the orange II adsorbed in the hydrogel was 0.106 x $10^{-4}$ (mol/g of polymer).

Change with time in the amount of the orange II released from the hydrogel adsorbing orange II therein

The hydrogel which was subjected to measurement of the amount of the orange II adsorbed as mentioned above was immersed in 20 ml of physiological saline at 37°C, after which samples of the immersion solution were taken at various intervals (1, 2, 3, 4, 5, 24, 48 and 72 hours), the light absorption at a wavelength of 486.8 nm was measured of each of the samples to determine the orange II content of the immersion solution. Thus, the total amount of orange II released from the hydrogel (accumulated amount of the orange II released) was measured, and percentage for the above amount of the orange II adsorbed was determined as the rate of the orange II released from the hydrogel.

The rate of the orange II released from the hydrogel was the following.

| Time elapsed | Release rate |
|---|---|
| 1 hr. | 5.1% |
| 2 hrs. | 9.3% |
| 3 hrs. | 12.9% |
| 4 hrs. | 16.6% |
| 5 hrs. | 18.9% |
| 24 hrs. | 44.1% |
| 48 hrs. | 61.8% |
| 72 hrs. | 78.1% |

Examples 2-4 (Active substance-containing polymer gel A)

In the same manner as in Example 1, AIBN was added to monomer mixtures comprising HEMA, QBm and EDMA and the resulting mixtures were subjected to radical polymerization. Thus produced polymers were swelled by hydration to provide the hydrogels of Examples 2-4. The amounts of the monomers, cross-linking monomer and radical polymerization initiator are listed in Table 1.

Thereafter, the resulting hydrogels were subjected to the same treatment as in Example 1 to adsorb orange II in the hydrogels of the respective examples, and then the free orange II not combined with the cations in the hydrogels was eliminated.

Similarly in Example 1, measurement was made of the amount of the orange II adsorbed in the respective hydrogels and of the total amount of the orange II released from the respective hydrogels.

Table 2 shows the result of measurement of the amount of orange II adsorbed in the respective hydrogels.

Also the change with time in the rate of the orange II released from each hydrogel is shown in Fig. 1.

Comparative Example 1

In a 50 ml ampoule there were placed 30.2 g (0.238 mol ) of HEMA, 0.25 g (0.5 mol% of the total monomers) of EDMA, and 0.033 g (0.08 mol% of the total monomers) of AIBN, and the mixture was stirred for 1 hour in a nitrogen atmosphere. After the stirring the mixture was treated in the same manner as in Example 1 to prepare the hydrogel of Comparative Example 1. Thereafter, the hydrogel was subjected to the same treatment as in Example 1 to adsorb orange II in the hydrogel, and then the free orange II not combined with the cations in the hydrogel was eliminated.

Similarly in Example 1, measurement was made of the amount of the orange II adsorbed in the hydrogel and of the total amount of the orange II released from the hydrogel.

Table 2 shows the result of measurement of the amount of the orange II adsorbed in the hydrogel.

Also the change with time in the rate of the orange II released from the hydrogel is shown in Fig. 1.

Table 1: Monomer composition

| | HEMA (mol) | QBm (mol) | EDMA (mol%)* | AIBM (mol%)* | Monomer comp. ratio (F) |
|---|---|---|---|---|---|
| Examples | | | | | |
| 1: | 0.247 | 0.0025 | 0.5 | 0.08 | 0.01 |
| 2: | 0.247 | 0.0025 | 2.0 | 0.08 | 0.01 |
| 3: | 0.238 | 0.0012 | 0.5 | 0.08 | 0.005 |
| 4: | 0.238 | 0.0012 | 2.0 | 0.08 | 0.005 |
| Comparative Example | | | | | |
| 1: | 0.232 | 0 | 0.5 | 0.08 | 0 |

*: Mol% of the total monomers.

Table 2: Amount of orange II adsorbed

| | Monomer composition ratio (F) | Adsorbed amount (mol/g of polymer) |
|---|---|---|
| Examples | | |
| 1 | 0.01 | $0.106 \times 10^{-4}$ |
| 2 | 0.01 | $0.106 \times 10^{-4}$ |
| 3 | 0.005 | $0.959 \times 10^{-5}$ |
| 4 | 0.005 | $0.881 \times 10^{-5}$ |
| Comparative Example | | |
| 1 | 0 | $0.492 \times 10^{-7}$ |

As shown in Table 2, compared with the results of Comparative Example 1, each of the hydrogels of Examples 1-4 adsorbed more active substance, orange II.

In Comparative Example 1, the amount of the orange II adsorbed in the hydrogel was as small as $0.492 \times 10^{-7}$ (mol/g of polymer), and completely no release was observed in physiological saline as shown by the release rate curve of Fig. 1.

In contrast, significant sustained release was observed in all of the Examples 1-4 over a long period of time in order of 72 hours.

This significant sustained release as shown in Fig. 1 is believed to have resulted from the presence of the bonding due to the electrostatic interaction between the ammonium groups in the hydrogels and orange II.

Example 5 (Active substance-containing polymer gel A)

In the same manner as in Example 1, AIBN was added to a monomer mixture comprising HEMA, QBm and EDMA and the resulting mixture was subjected to radical polymerization. Thus produced polymer was swelled by hydration to provide the hydrogel of Example 5. The amounts of the monomers, cross-linking monomer and radical polymerization initiator are listed in Table 3. Next, to 10 ml of distilled water was added 3.2 mg (1 x $10^{-5}$ mol) of sodium guaiazulenesulfonate (hereunder abbrev. to water-soluble azulene), and in 10 ml of the resulting aqueous solution of the water-soluble azulene (1.0 x $10^{-3}$ (mol/l)) was immersed the above mentioned gel at 25°C for 24 hours to adsorb the water-soluble azulene in the hydrogel. Thereafter, the hydrogel with the water-soluble azulene adsorbed therein was immersed in 50 ml of distilled water at 25°C for 48 hours to eliminate the free water-soluble azulene not combined with the cations in the hydrogel.

Measurement of the amount of the water-soluble azulene adsorbed in the hydrogel

As mentioned above, the hydrogel was immersed in 10 ml of a solution of water-soluble azulene in ethanol (1.0 x $10^{-3}$ mol/l) for 24 hours; and the adsorption was confirmed to have attained the equilibrium. Thereafter, in order to measure the amount of the water-soluble azulene adsorbed in the hydrogel, measurement of light absorption at a wavelength of 486.6 nm was made of the residual solution of the water-soluble azulene in ethanol after the 24 hour immersion (liquid C) and of the distilled water which contained the free water-soluble azulene released from the water-soluble azulene-containing hydrogel after immersing in the distilled water for 48 hours (liquid D), to determine the water-soluble azulene content each of the liquids C and D. The difference was calculated to determine the amount of the water-soluble azulene combined with the cations in the above hydrogel. The spectrophotometer used was Model U-3210 of Hitachi Ltd. in Japan .

The amount of the water-soluble azulene adsorbed in the hydrogel of this example was 0.343 x $10^{-4}$ - (mol/g of polymer).

Change with time in the amount of the water-soluble azulene released from the hydrogel adsorbing water-soluble azulene therein

The hydrogel which was subjected to measurement of the amount of the water-soluble azulene adsorbed as mentioned above was immersed in 20 ml of physiological saline at 37°C, after which samples of the immersion solution were taken at various intervals (1, 2, 3, 4, 5, 24, 48 and 72 hours), the light absorption at a wavelength of 292.8 nm was measured of each of the samples to determine the water-soluble azulene content of the immersion solution. Thus the total amount of water-soluble azulene released from the hydrogel (accumulated release amount) was measured , and percentage for the above amount adsorbed was determined as the rate of the water-soluble azulene released from the hydrogel.

The rate of the water-soluble azulene released from the hydrogel was the following.

| Time elapsed | Release rate |
|---|---|
| 1 hr. | 16.3% |
| 2 hrs. | 28.9% |
| 3 hrs. | 38.4% |
| 4 hrs. | 45.7% |
| 5 hrs. | 53.1% |
| 24 hrs. | 82.6% |
| 48 hrs. | 92.6% |
| 72 hrs. | 95.4% |

Examples 6-8 (Active substance-containing polymer gel A)

In the same manner as in Example 1, AIBN was added to monomer mixtures comprising HEMA, QBm and EDMA and the resulting mixtures were subjected to radical polymerization. Thus produced polymers were swelled by hydration to provide the respective hydrogels of Examples 6-8. The amounts of the monomers, cross-linking monomer and radical polymerization initiator are listed in Table 3.

Thereafter, the resulting hydrogels were subjected to the same treatment as in Example 5 to adsorb the water-soluble azulene in the hydrogels of the respective examples, and then the free water-soluble azulene not combined with the cations in the hydrogels was eliminated.

Similarly in Example 5, measurement was made of the amount of the water-soluble azulene adsorbed in the respective hydrogels and of the total amount of the water-soluble azulene released from the respective hydrogels.

Table 4 shows the result of measurement of the amount of the water-soluble azulene adsorbed in the respective hydrogels.

Also the change with time in the rate of the water-soluble azulene released from each hydrogel is shown in Fig. 2.

Comparative Example 2

In a 50 ml ampoule there were placed 30.2 g (0.238 mol ) of HEMA, 0.25 g (0.5 mol % of the total monomers) of EDMA, and 0.033 g (0.087 mol % of the total monomers) of AIBN, and the mixture was stirred for 1 hour in a nitrogen atmosphere. After the stirring the mixture was treated in the same manner as in Example 5 to prepare the hydrogel of Comparative Example 2. Thereafter, the hydrogel was subjected to the same treatment as in Example 5 to adsorb the water-soluble azulene in the hydrogel, and then the free water-soluble azulene not combined with the cations in the hydrogel was eliminated.

Similarly in Example 5, measurement was made of the amount of the water-soluble azulene adsorbed in the hydrogel and of the total of the water-soluble azulene released from the hydrogel.

Table 4 shows the result of measurement of the amount of the water-soluble azulene adsorbed in the hydrogel.

Also the change with time in the rate of the water-soluble azulene released from the hydrogel is shown in Fig. 2.

Table 3: Monomer composition

| | HEMA (mol) | QBm (mol) | EDMA (mol%)* | AIBM (mol%)* | Monomer comp. ratio (F) |
|---|---|---|---|---|---|
| Examples | | | | | |
| 5: | 0.247 | 0.0025 | 0.5 | 0.08 | 0.01 |
| 6: | 0.247 | 0.0025 | 2.0 | 0.08 | 0.01 |
| 7: | 0.238 | 0.0012 | 0.5 | 0.08 | 0.005 |
| 8: | 0.238 | 0.0012 | 2.0 | 0.08 | 0.005 |
| Comparative Example | | | | | |
| 2: | 0.232 | 0 | 0.5 | 0.08 | 0 |

*: Mol % of the total monomers.

Table 4: Amount of water-soluble azulene adsorbed

| | | Monomer composition ratio (F) | Adsorbed amount (mol/g of polymer) |
|---|---|---|---|
| Examples | | | |
| | 5 | 0.01 | $0.343 \times 10^{-4}$ |
| | 6 | 0.01 | $0.286 \times 10^{-4}$ |
| | 7 | 0.005 | $0.650 \times 10^{-5}$ |
| | 8 | 0.005 | $0.738 \times 10^{-5}$ |
| Comparative Example | 2 | 0 | $0.219 \times 10^{-5}$ |

As shown in Table 4, compared with the results of Comparative Example 2, each of the hydrogels of Examples 5-8 adsorbed more active substance, water-soluble azulene.

In Comparative Example 2, the amount of the water-soluble azulene adsorbed in the hydrogel was as small as $0.219 \times 10^{-5}$ (mol/g of polymer), and completely no release was observed in physiological saline as shown by the release rate curve of Fig. 2.

In contrast, significant sustained release was observed in all the Examples 5-8 over a long period of time in the order of 72 hours.

This significant sustained release as shown in Fig. 2 is believed to have resulted from the presence of the bonding due to the electrostatic interaction between the ammonium groups in the hydrogels and water-soluble azulene.

Example 9 (Active substance-containing polymer gel B)

In a 50 ml ampoule there were placed 23.7 g (0.182 mol ) of HEMA, 2 g (0.02 mol ) of methyl methacrylate (hereunder abbrev. to MMA), 0.42 g (0.002 mol ) of vinylbenzyl trimethyl ammonium salt (hereunder abbrev. as QBm), 0.202 g (0.5 mol % of the total monomers) of EDMA, and 0.027 g (0.08 mol % of the total monomers) of AIBN, and the mixture was stirred for 1 hour in a nitrogen atmosphere. Here, the monomeric composition ratio F was 0.01. After stirring, the mixture was transferred to a polyethylene container (d.: 15 mm, h.: 17 mm), and then subjected to polymerization at 50-100 °C for 72 hours. The resulting polymer was taken out of the container and cut into flat disks with a diameter of 13 mm and a thickness of 0.5 mm, after which the surface was polished to provide flat transparent disks. The disks were immersed in distilled water at 80 °C for 2 hours for swelling due to hydration, thereby producing a hydrogel from which the residual monomers were removed.

Separately, 5.12 mg ($1 \times 10^{-5}$ mol) of disodium cromoglycate (hereunder abbrev. to DSCG), an active substance, was added to 10 ml of distilled water, followed by mixing and stirring, to prepare 10 ml of an aqueous solution of DSCG in which the above mentioned hydrogel was placed for a 24 hours immersion therein at 25 °C to adsorb the DSCG in the hydrogel. Thereafter, the hydrogel with the DSCG adsorbed therein was immersed into 50 ml of distilled water at 37 °C for 48 hours to eliminate the free DSCG not combined with the cations in the hydrogel.

Measurement of linear swelling coefficient and water content of polymer

A polymer material having a diameter of 13 mm and a thickness of 0.5 mm when dried was immersed in distilled water at 20 °C for 48 hours; the swelling was confirmed to have attained the equilibrium. Then

measurement of the polymer was made of the diameter prior to hydration swelling (Dd), that after hydration swelling (Dw), the weight after hydration swelling (Wω) and that after redrying (Wd), then calculation was made of the linear swelling coefficient (hereunder referred to as "swelling coefficient") and the water content.

Swelling coefficient = [(Dw - Dd) x 100]/ Dd
Water content = [(Wω - Wd) x 100]/ Wω

Measurement of the amount of the DSCG adsorbed in the hydrogel

As mentioned above, the hydrogel was immersed in 10 ml of an aqueous solution of DSCG (1.0 x $10^{-3}$ mol/l) for 24 hours; the adsorption was confirmed to have attained the equilibrium. Thereafter, in order to measure the amount of the DSCG adsorbed in the hydrogel, measurement of the light absorption at a wavelength of 239 nm was made of the residual aqueous solution of DSCG after the 24 hours immersion (liquid A) and of the distilled water which contained the free DSCG released from the DSCG-adsorbing hydrogel immersed in distilled water for 48 hours (liquid B) to determine the DSCG content each of the liquids A and B. The difference was calculated to determine the amount of the DSCG combined with the cations in the above hydrogel. Here, the spectrophotometer used was Model U-3210 of Hitachi Ltd. in Japan).

The amount of the DSCG adsorbed on the hydrogel of this example was 0.218 x $10^{-4}$ (mol/g of polymer).

Change with time in the amount of the DSCG released from the hydrogel adsorbing DSCG therein

The hydrogel which was subjected to measurement of the amount of the DSCG adsorbed as mentioned above was immersed in 20 ml of physiological saline at 37°C, after which samples of the immersion solution were taken at various intervals (1, 2, 3, 4, 5, 24, 48 and 72 hours), the light absorption at a wavelength of 239 nm was measured of each of the samples to determine the DSCG content of the immersion solution. Thus the total amount of DSCG released from the hydrogel (accumulated amount of the DSCG released) was measured, and percentage for the above amount of the DSCG adsorbed was determined as the rate of the DSCG released from the hydrogel.

The release rate of the DSCG released from the hydrogel was the following.

| Time elapsed | Release rate |
|---|---|
| 1 hr. | 23.2% |
| 2 hrs. | 31.8% |
| 3 hrs. | 38.4% |
| 4 hrs. | 44.6% |
| 5 hrs. | 49.6% |
| 24 hrs. | 84.0% |
| 48 hrs. | 94.1% |
| 72 hrs. | 97.8% |

Example 10 (Active substance-containing polymer gel B)

In the same manner as in Example 9, AIBN was added to a monomer mixture comprising HEMA, MMA and EDMA for radical polymerization. The resulting polymer was swelled by hydration to provide a hydrogel. The amounts of the monomers, cross-linking monomer and radical polymerization initiator are listed in Table 5.

Thereafter, the resulting hydrogel was subjected to the same treatment as in Example 9 to adsorb the DSCG in the hydrogel, and then the free DSCG not combined with the cations in the hydrogel were eliminated.

Similarly in Example 9, measurement was made of the amount of the DSCG adsorbed in the hydrogel and of the total amount of DSCG released from the respective hydrogel.

Table 2 shows the result of measurement of the swelling coefficient and water content of the polymer and the amount of the DSCG adsorbed in the hydrogel.

Also the change with time in the rate of the DSCG released from each hydrogel is shown in Fig. 3.

Examples 11-12 (Active substance-containing polymer gel B)

In the same manner as in Example 9, AIBN was added to a monomer mixture comprising HEMA, n-butyl methacrylate (hereunder abbrev. to BMA), QBm and EDMA for radical polymerization. The resulting polymer was swelled by hydration to provide the respective hydrogels of Examples 11-12. The amounts of the monomers, cross-linking monomer and radical polymerization initiator are listed in Table 5.

Thereafter, the resulting hydrogels were subjected to the same treatment as in Example 9 to adsorb the DSCG in the hydrogels of the respective examples, and then the free DSCG not combined with the cations in the hydrogels were eliminated.

Similarly in Example 9, measurement was made of the amount of the DSCG adsorbed in the respective hydrogels and of the total amount of DSCG released from the respective hydrogels.

Table 6 shows the result of measurement of the swelling coefficient and water content of the polymers and of the amount of the DSCG adsorbed in the hydrogels.

Also the change with time in the rate of the DSCG released from each hydrogels is shown in Fig. 4.

Examples 13-14 (Active substance-containing polymer gel B)

In the same manner as in Example 9, AIBN was added to a monomer mixture comprising HEMA, 2,2,2-trifluoroethyl methacrylate (hereunder abbrev. to 3FE), QBm and EDMA for radical polymerization. The resulting polymer was swelled by hydration to provide the respective hydrogels of Examples 13-14. The amounts of the monomers, cross-linking monomer and radical polymerization initiator are listed in Table 5.

Thereafter, the resulting hydrogels were subjected to the same treatment as in Example 9 to adsorb the DSCG in the hydrogels of the respective examples, and then the free DSCG not combined with the cations in the hydrogels were eliminated.

Similarly in Example 9, measurement was made of the amount of the DSCG adsorbed in the respective hydrogels and of the total amount of DSCG released from the respective hydrogels.

Table 6 shows the result of measurement of the swelling coefficient and water content of the polymers and of the amount of the DSCG adsorbed in the hydrogels.

Also the change with time in the rate of the DSCG released from each hydrogels is shown in Fig. 5.

Table 5: Monomer composition

| Examples | HEMA (mol) | MMA (mol) | BMA (mol) | 3FE (mol) | QBm (mol) | EDMA (mol%)* | AIBN (mol%)* | Monomer comp. ratio (F) |
|---|---|---|---|---|---|---|---|---|
| 9 | 0.182 | 0.02 | 0 | 0 | 0.002 | 0.5 | 0.08 | 0.01 |
| 10 | 0.167 | 0.04 | 0 | 0 | 0.002 | 0.5 | 0.08 | 0.01 |
| 11 | 0.182 | 0 | 0.014 | 0 | 0.002 | 0.5 | 0.08 | 0.01 |
| 12 | 0.167 | 0 | 0.028 | 0 | 0.002 | 0.5 | 0.08 | 0.01 |
| 13 | 0.182 | 0 | 0 | 0.012 | 0.002 | 0.5 | 0.08 | 0.01 |
| 14 | 0.167 | 0 | 0 | 0.024 | 0.002 | 0.5 | 0.08 | 0.01 |
| 1 | 0.198 | 0 | 0 | 0 | 0.002 | 0.5 | 0.08 | 0.01 |

* Mol % of the total monomers.

In the table,
* Mol% of the totalmonomers.
HEMA: 2-hydroxyethyl methacrylate;
MMA: methyl methacrylate;
BMA: n-butyl methacrylate;
3FE: 2,2,2-trifluoroethyl methacrylate;

QBM: vinylbenzyl trimethyl ammonium salt;
EDMA: ethylene glycol dimethacrylate (cross-linking monomer); and
AIBN: azobisisobutyronitrile (radical polymerization initiator).

Table 6

| Results of measurement of swelling coefficient, water content and DSCG adsorption amount of the polymers | | | |
|---|---|---|---|
| Examples | Swelling coefficient(%) | Water content(%) | DSCG adsorption amount (mol/g of polymer) |
| 9 | 17.2 | 34.5 | $0.228 \times 10^{-4}$ |
| 10 | 14.3 | 30.1 | $0.219 \times 10^{-4}$ |
| 11 | 15.2 | 31.1 | $0.254 \times 10^{-4}$ |
| 12 | 11.1 | 25.4 | $0.191 \times 10^{-4}$ |
| 13 | 15.5 | 32.0 | $0.245 \times 10^{-4}$ |
| 14 | 12.1 | 25.7 | $0.191 \times 10^{-4}$ |
| 1 | 21.1 | 39.1 | $0.198 \times 10^{-4}$ |

In the table, DSCG represents disodium cromoglycate (active substance).

As shown in Table 6, compared with active substance-containing polymer gel A of Example 1, almost no difference in the DSCG adsorption amount was found for the active substance-containing polymer gel B of Examples 9-14, though all of them underwent lowered swelling coefficient and water content.

Relating to the hydrogel of Example 1, most of the DSCG adsorbed in the hydrogel was released in 24 hours as shown by the release rate curves in Figs. 3-5.

In contrast, significant sustained release was observed in all the Examples 9-14 over a long period of time in the order of 72 hours.

This significant sustained release as shown in Figs. 3-5 is believed to have resulted from the introduction of R(M)A into the hydrogels thereby reducing the diffusion rate of the DSCG in the hydrogels.

As mentioned above, according to the present invention it is possible to produce active substance-containing polymer gel A capable of strongly holding an anionic functional group-containing active substance therein to produce a significant sustained release effect and of being worked into any desired form in an extremely easy manner.

Also according to the present invention it is possible to produce active substance-containing polymer gel B capable of strongly holding an anionic functional group-having active substance therein to produce a significant sustained release effect by controlling the swelling coefficient and water content of the hydrogel.

Accordingly, active substance-containing polymer gels A and B of the present invention may be employed for the preparation of various drug delivery systems (DDS), fungicidal sheets, insecticidal sheets, fomentation, etc with advantages, and particularly preferably for the preparation of ophthalmic insertions.

**Claims**

**1.** An active substance-containing polymer gel characterized by comprising an active substance with an anionic substituent thereon, held by a copolymer gel through adsorption therein, said copolymer gel being prepared by copolymerization of a monomer mixture containing at least a hydrocarbon group-containing (meth)acrylate which has one or more hydroxyl groups and has optionally an intramolecular ether linkage; and a monomer with a quaternary ammonium salt on the side chain.

**2.** The active substance-containing polymer gel according to Claim 1, wherein the hydrocarbon group-containing (meth) acrylate which has one or more hydroxyl groups and has optionally an intramolecular ether linkage is 2-hydroxyehtyl methacrylate.

**3.** The active substance-containing polymer gel according to Claim 1, wherein the monomer with a quaternary ammonium salt on the side chain is vinylbenzyl trialkyl ammonium salt represented by the following general formula (I):

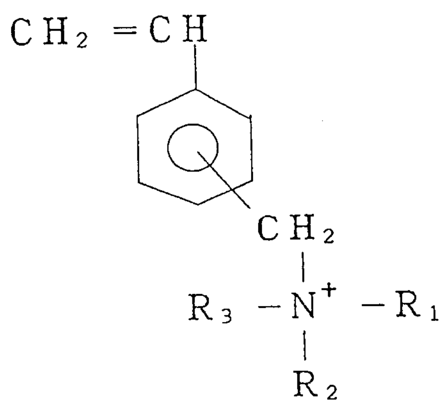

(I)

wherein $R_1$ is a $C_5$-$C_{12}$ alkyl group, $R_2$ and $R_3$ are the same or different $C_1$-$C_2$ alkyl groups, or $R_1$, $R_2$ and $R_3$ are the same or different $C_1$-$C_4$ alkyl groups.

4. The active substance-containing polymer gel according to Claim 1, wherein the monomer with a quaternary ammonium salt on the side chain is ethyl methacrylate represented by the following general formula (II):

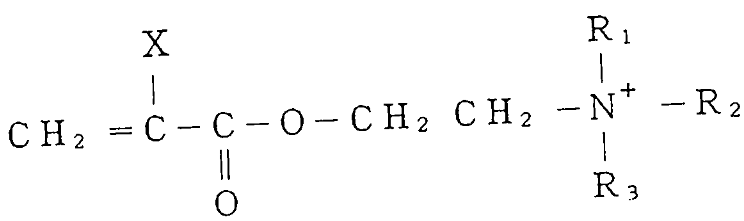

(II)

wherein X is a methyl group or a hydrogen atom, $R_1$ is a $C_5$-$C_{12}$ alkyl group, $R_2$ and $R_3$ are the same or different $C_1$-$C_2$ alkyl groups, or $R_1$, $R_2$ and $R_3$ are the same or different $C_1$-$C_4$ alkyl groups.

5. The active substance-containing polymer gel according to Claim 1, wherein the active substance with an anionic substituent thereon is an organic compound which has at least one intramolecular anionic substituent selected from a group consisting of carboxylic acid group, sulfonic acid group, phenolic hydroxy group and salts thereof.

6. The active substance-containing polymer gel according to Claim 1, wherein the monomer mixture contains at least one additional crosslinkable monomer.

7. The active substance-containing polymer gel according to any of Claims 1-6, which is used for an ophthalmic application.

8. An active substance-containing polymer gel characterized by comprising an active substance with an anionic substituent therein, held by a copolymer gel through adsorption therein, said copolymer gel being prepared by copolymerization of a monomer mixture containing at least a hydrocarbon group-containing (meth)acrylate which has one or more hydroxyl groups and has optionally an intramolecular ether linkage; and a monomer with a quaternary ammonium salt on the side chain; a (meth) acrylate having an alkyl or fluoroalkyl group.

9. The active substance-containing polymer gel according to Claim 8, wherein the hydrocarbon group-containing (meth) acrylate which has one or more hydroxyl groups and has optionally an intramolecular ether linkage is 2-hydroxyehtyl methacrylate.

**10.** The active substance-containing polymer gel according to Claim 8, wherein the monomer having an alkyl or fluoroalkyl group is a methacrylate represented by the following general formula (III):

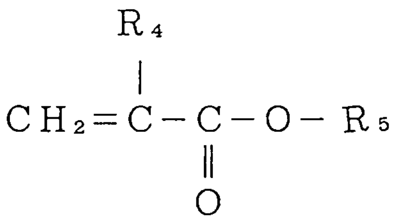

(III)

wherein $R^4$ is a methyl group or a hydrogen atom, $R_5$ is a hydrogen atom or a $C_1$-$C_{12}$ alkyl group which may be substituted with a fluorine atom.

**11.** The active substance-containing polymer gel according to Claim 8, wherein the monomer with a quaternary ammonium salt on the side chain is vinylbenzyl trialkyl ammonium salt represented by the following general formula (I):

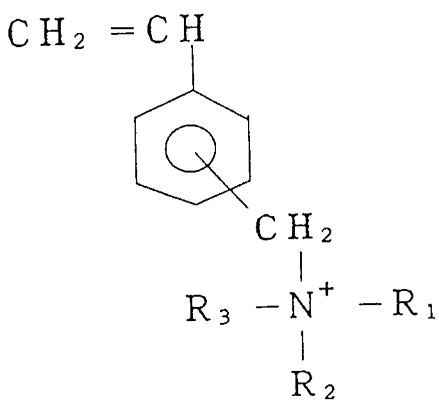

(I)

wherein $R_1$ is a $C_5$-$C_{12}$ alkyl group, $R_2$ and $R_3$ are the same or different $C_1$-$C_2$ alkyl groups, or $R_1$, $R_2$ and $R_3$ are the same or different $C_1$-$C_4$ alkyl groups.

**12.** The active substance-containing polymer gel according to Claim 8, wherein the monomer with a quaternary ammonium salt on the side chain is ethyl methacrylate represented by the following general formula (II):

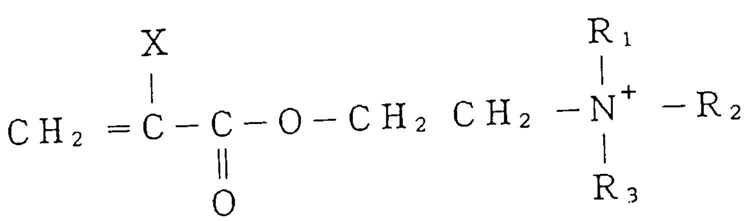

(II)

wherein X is a methyl group or a hydrogen atom, $R_1$ is a $C_5$-$C_{12}$ alkyl group, $R_2$ and $R_3$ are the same or different $C_1$-$C_2$ alkyl groups, or $R_1$, $R_2$ and $R_3$ are the same or different $C_1$-$C_4$ alkyl groups.

**13.** The active substance-containing polymer gel according to Claim 8, wherein the active substance with an anionic substituent thereon is an organic compound which has at least one intramolecular anionic substituent selected from a group consisting of carboxylic acid group, sulfonic acid group, phenolic hydroxy group and salts thereof.

**14.** The active substance-containing polymer gel according to Claim 8, wherein the monomer mixture contains at least one additional crosslinkable monomer.

**15.** The active substance-containing polymer gel according to any of Claims 8-14, which is used for an ophthalmic application.

FIG. I

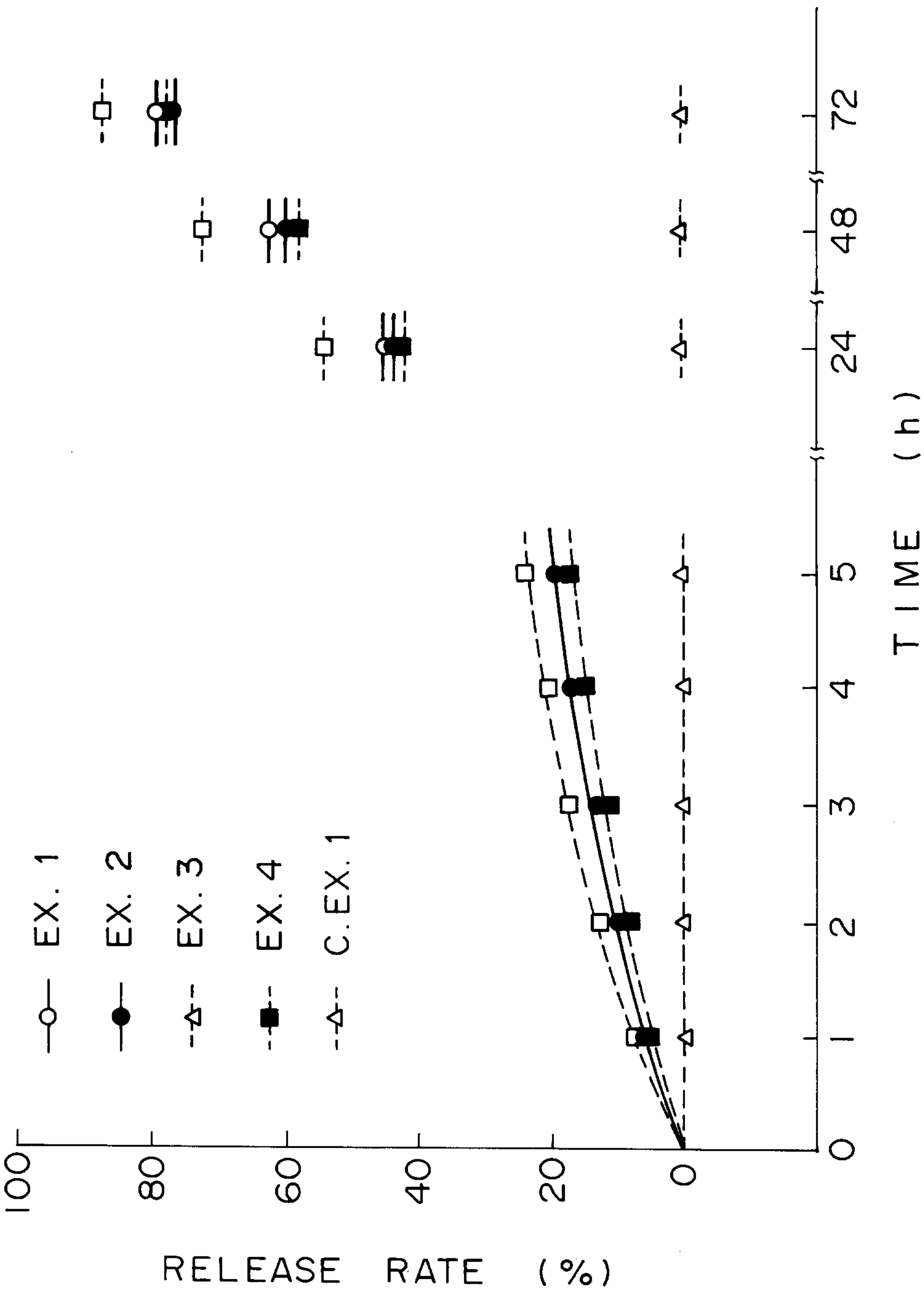
EX. 1
EX. 2
EX. 3
EX. 4
C.EX. 1
RELEASE RATE (%)
100
80
60
40
20
0
TIME (h)
0
1
2
3
4
5
24
48
72

FIG. 2

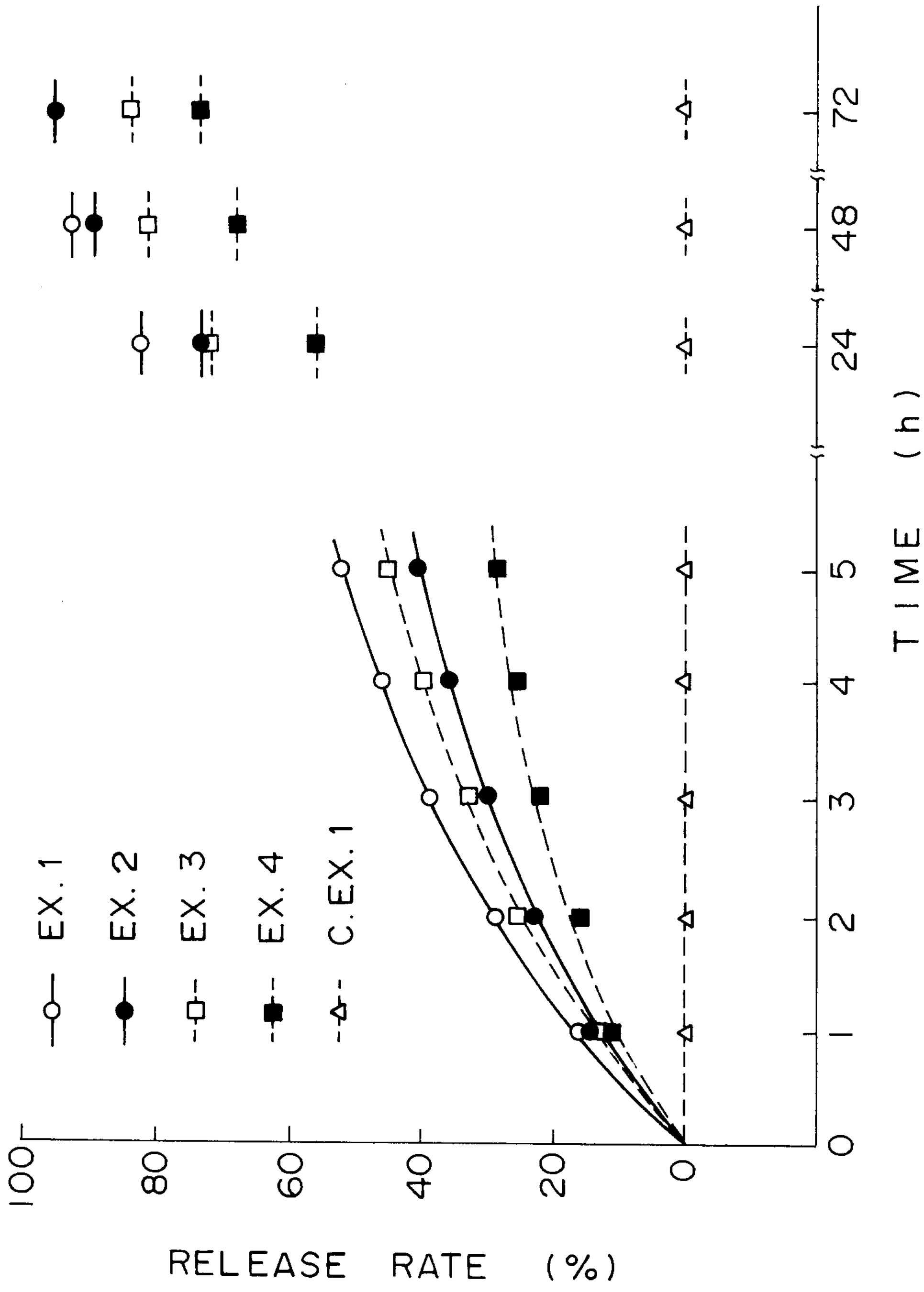
EX. 1
EX. 2
EX. 3
EX. 4
C. EX. 1
RELEASE RATE (%)
100
80
60
40
20
0
0
1
2
3
4
5
24
48
72
TIME (h)

FIG. 3

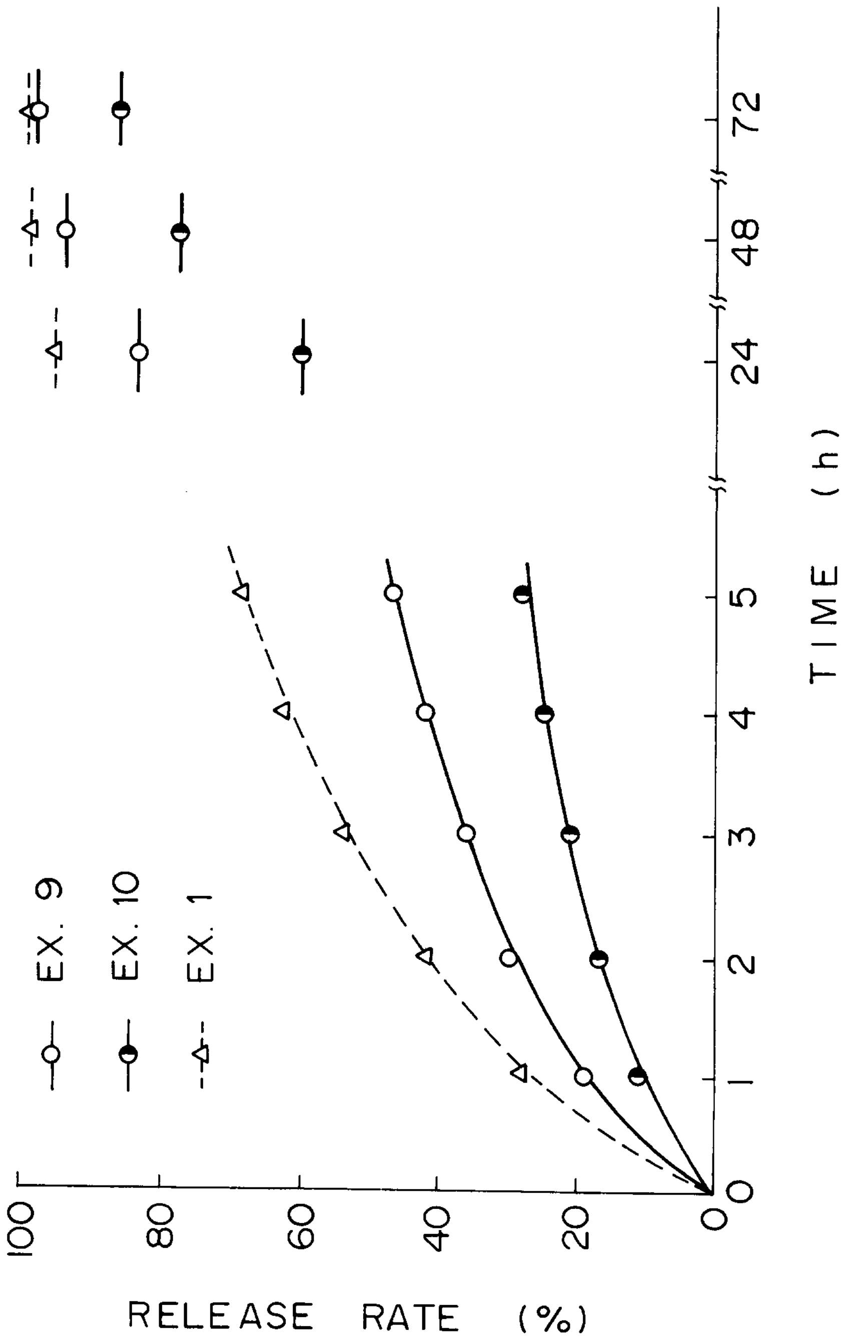
RELEASE RATE (%)
100
80
60
40
20
0
0
1
2
3
4
5
24
48
72
TIME (h)
EX. 9
EX. 10
EX. 1

FIG. 4

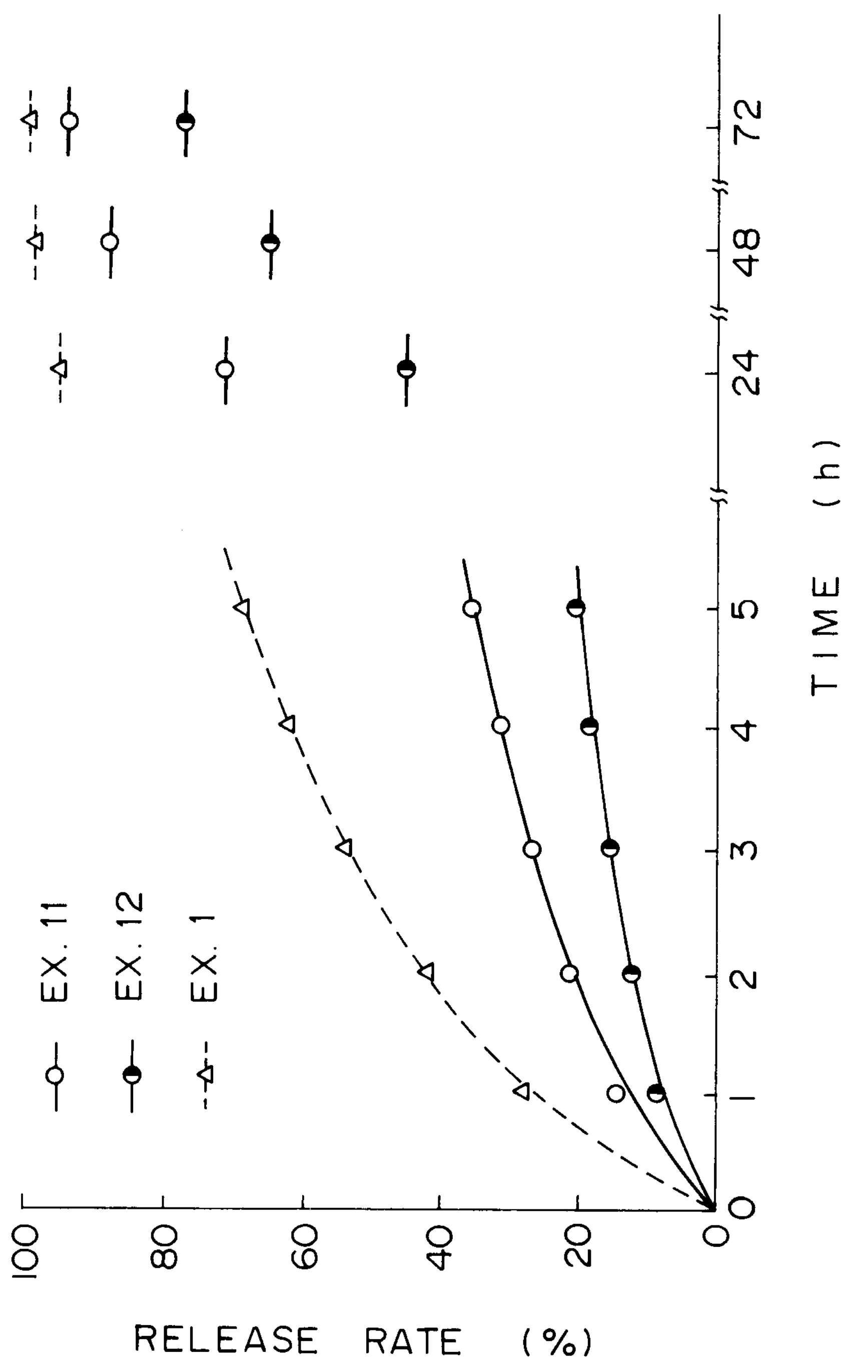
EX. 11
EX. 12
EX. 1
RELEASE RATE (%)
100
80
60
40
20
0
0
1
2
3
4
5
24
48
72
TIME (h)

FIG. 5

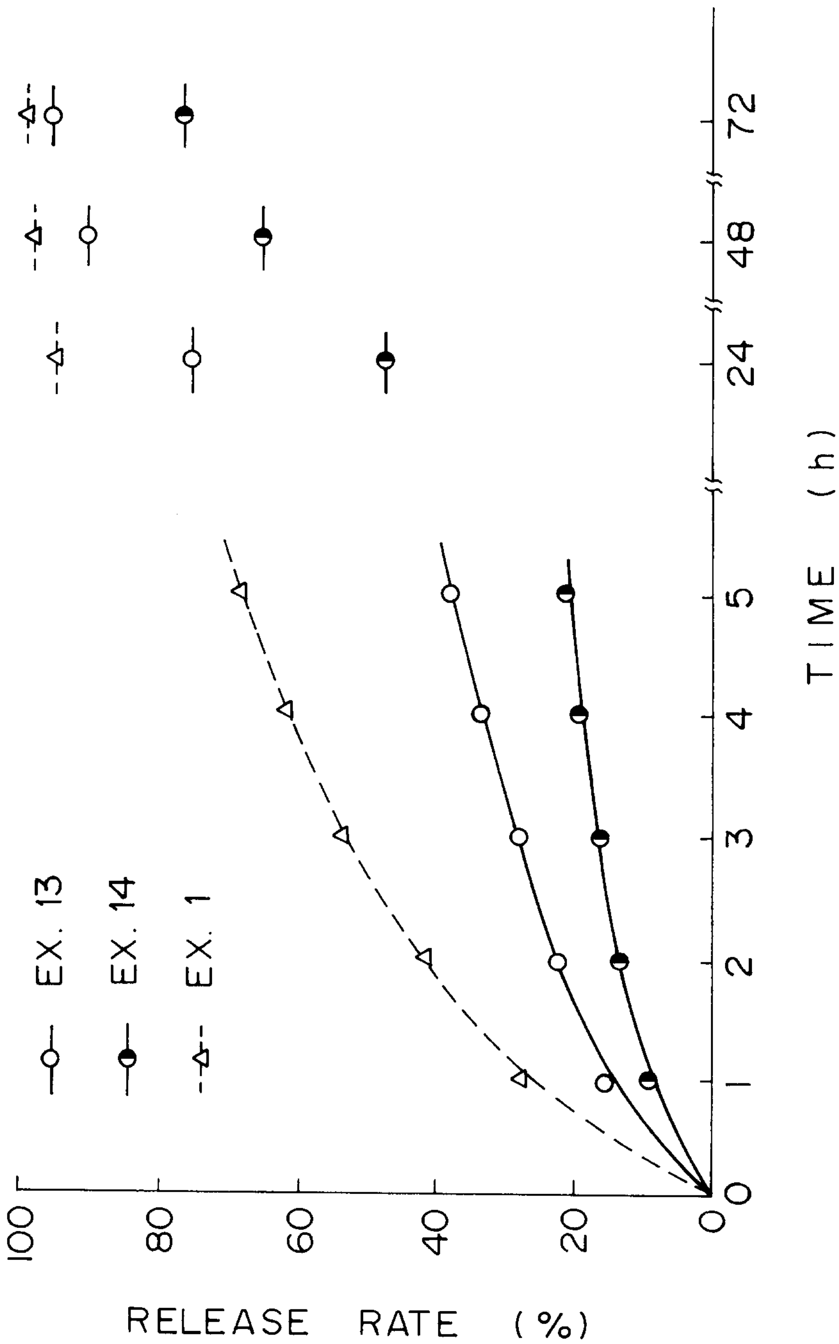
EX. 13
EX. 14
EX. 1
RELEASE RATE (%)
100
80
60
40
20
0
TIME (h)
0
1
2
3
4
5
24
48
72